# FASCICULE DE BREVET EUROPEEN

(11) **EP 3 618 743 B1**
(45) Date de publication et mention de la délivrance du brevet: **19.02.2025**
(21) Numéro de dépôt: 18721047.1
(22) Date de dépôt: 04.05.2018
(51) Int. Cl.: A61B 17/70, A61B 17/88

(54) **ANCILLAIRE DE MISE EN TENSION D'UN ELEMENT LONGILIGNE**
ZUBEHÖR ZUM SPANNEN EINES LÄNGLICHEN ELEMENTS
ACCESSORY FOR TENSIONING AN ELONGATE ELEMENT

(30) Priorité: 04.05.2017 FR 1753949
(43) Date de publication de la demande: 11.03.2020
(73) Titulaire: Cousin Biotech, 59117 Wervicq Sud (FR)
(72) Inventeur: DENEUVILLERS, Guy, 62155 MERLIMONT (FR); PRANDI, Jules, 59200 TOURCOING (FR); KAMCHE, Farid, 59200 TOURCOING (FR)
(74) Mandataire: Cabinet Beau de Loménie
(86) Numéro de dépôt international: PCT/EP2018/061478
(87) Numéro de publication internationale: WO 2018/202839

(56) Documents cités:
- EP-A1- 0 019 062
- US-A- 5 868 748
- US-A1- 2010 030 240
- US-A1- 2011 087 225

## Description

### Arrière-plan de l'invention

La présente invention concerne le domaine technique des ancillaires de mise en tension d'un élément longiligne, notamment plat, pour la fixation d'un implant sur un élément osseux, notamment au moins une portion d'un corps vertébral.

Il est connu de corriger les courbures du rachis anormales et/ou les inclinaisons pathologiques des vertèbres entre-elles par l'utilisation de barres longitudinales implantables disposées de part et d'autre de la colonne vertébrale et solidarisées aux vertèbres par l'intermédiaire de vis insérées dans les vertèbres elles-mêmes ou de crochets, introduits par exemple le long du canal rachidien. Ces vis et crochets sont cependant agressifs pour le rachis.

Pour pallier à ces inconvénients, il a été proposé un élément longiligne flexible de fixation maintenu en tension par l'intermédiaire d'un implant, lequel implant est éventuellement configuré pour être solidarisé à une barre longitudinale, l'élément longiligne fixé à l'implant venant en prise avec au moins une portion vertébrale en formant une boucle autour de cette dernière.

Les barres longitudinales implantables peuvent être mises en œuvre dans le traitement de la scoliose ou encore le traitement de pathologies dégénératives.

On rencontre différents niveaux de dégénérescence, par exemple sur un niveau vertébral telle qu'une discopathie ou sur deux niveaux vertébraux tel que le spondylolisthésis ou encore plus de trois niveaux comme la scoliose en C ou en S.

Dans ces traitements, la barre longitudinale peut être mise en liaison avec le niveau vertébral à corriger par l'intermédiaire d'un élément longiligne, lequel passe autour au moins d'une portion d'un corps vertébral et autour de ladite barre puis les extrémités dudit élément longiligne peuvent être maintenues au moyen d'une suture, d'un nœud ou à l'aide d'un moyen de solidarisation implantable. Ledit moyen de solidarisation peut comprendre un logement recevant la barre longitudinale et l'élément longiligne à l'état enroulé autour de cette dernière, et des moyens de serrage pour le blocage de la barre et dudit élément longiligne dans ledit logement. Généralement, les moyens de serrage comprennent des moyens de pincement dudit élément longiligne empêchant tout mouvement de ce dernier. Il est également possible de ne pas utiliser une barre longitudinale implantable mais uniquement un élément longiligne passant autour au moins une portion d'un corps vertébral, et notamment sur un ou plusieurs niveau(x) vertébral/aux, ledit élément longiligne étant maintenu en tension par l'intermédiaire d'un implant solidarisant ses deux extrémités ensembles.

L'un des problèmes que cherche à résoudre la présente invention est de mettre en tension l'élément longiligne lorsqu'il forme une boucle sur au moins une portion d'un corps vertébral, et est en prise avec un implant et/ou une barre longitudinale implantable.

EP 1.933.743 B1 a pour objet un ancillaire de mise en tension d'un élément longiligne comprenant une pièce cylindrique mobile apte à se déplacer en translation autour d'une tige, ladite tige comprenant à son extrémité distale des moyens d'appui sur une barre longitudinale. La pièce cylindrique mobile comprend également un ergot pour la fixation de l'élément longiligne. Le système de serrage dynamométrique est actionnable par l'intermédiaire d'une poignée. La mise en tension de l'élément longiligne est effectuée par le chirurgien en pressant la poignée sans limite de la tension qui peut être appliquée. Le chirurgien peut ainsi appliquer une tension trop importante sur l'élément longiligne, ce qui risque de nuire au réglage de la ou des barres longitudinale(s) placée(s) sur le ou les niveau(x) vertébral(aux) à corriger, voir même de déplacer des vertèbres. En outre, la tension est appliquée sur une boucle dans le prolongement des deux extrémités libres de l'élément longiligne solidarisées à l'aide d'un passant. La tension appliquée ne sera donc pas la même sur chacune des extrémités libres puisqu'elle est appliquée directement sur une seule extrémité libre de l'élément longiligne. L'application de la tension est donc déséquilibrée sur chaque extrémité libre de l'élément longiligne et risque d'entraîner l'érosion de la portion vertébrale sur laquelle la tension est appliquée et/ou le glissement de l'élément longiligne sur ladite portion vertébrale.

FR 2.981.841 A1 a pour objet un ancillaire de mise en tension cherchant à éviter comparativement à EP 1.933.743 B1 que l'élément longiligne ne soit vrillé lorsqu'il est mis en tension. L'ancillaire comprend ainsi un renvoi afin que l'élément longiligne forme un angle de 90°. Les extrémités de l'élément longiligne munies de moyens de solidarisation sont fixées à une roue de compensation selon deux points diamétralement opposés. La tension appliquée sur chacune des extrémités de l'élément longiligne n'est donc pas équilibrée. En outre, le renvoi selon un angle de 90° de l'élément longiligne ainsi que le moyen de fixation d'une extrémité dans une saignée engendrent des frottements importants sur l'élément longiligne risquant de le fragiliser. De plus, du fait de l'organe de renvoi, l'ancillaire est encombrant. Or, lors d'une intervention chirurgicale, de multiples implants de maintien et barres longitudinales sont placés sur le ou les niveaux vertébral(aux) à corriger, de sorte qu'il est important pour le chirurgien, une fois un élément longiligne mis en tension, de pouvoir accéder facilement à l'implant de maintien de l'élément longiligne pour terminer le serrage et la fixation de ce dernier sur l'élément longiligne mais également de pouvoir accéder aux autres implants de maintien et/ou barres longitudinales implantables.

WO 2016/051088 a pour objet un ancillaire de mise en tension comprenant un chariot mobile monté sur un arbre rotatif disposé autour de l'axe longitudinal du corps de l'ancillaire, la mise en rotation de l'arbre entrainant en translation le chariot en liaison avec l'élément longiligne.

Dans les documents précités, la mise tension est obtenue par déplacement d'un élément mobile, en liaison avec l'élément longiligne, en translation le long d'un axe longitudinal. Ces ancillaires de mise en tension comprennent des dispositifs de mise en tension complexes et volumineux notamment du fait de l'encombrement de la course de serrage selon laquelle translate l'élément mobile.

US 5.868.748 a pour objet un appareil et une méthode pour serrer et pincer un câble chirurgical utilisé à des fins orthopédiques. L'appareil inclut une paire de poignées qui sont poussées l'une vers l'autre, et qui opèrent des mors opposés auxquels elles sont connectées. Une paire de cabestans est disposée sur des côtés opposés des poignées. Les mors sont adaptés pour maintenir un membre de pincement entre-eux sans le déformer tandis que les extrémités opposées d'une longueur de câble sont passées à travers le membre de pincement dans des directions opposées. Chaque extrémité du câble est enroulée autour d'un cabestan dédié. Une poignée à cliquet fait tourner simultanément les deux cabestans pour enrouler les extrémités opposées du câble. Lorsque la force de traction désirée est atteinte, les mors sont activés pour pincer le membre de serrage.

US 2011/0087225 A1 a pour objet un outil orthopédique comportant un palier de verrouillage unidirectionnel et un palier d'actionnement unidirectionnel pour tendre des câbles chirurgicaux autour d'un os, ainsi qu'une méthode d'utilisation de cet outil.

US2010030240A1 a pour objet un appareil pour lier ensemble deux parties de tissu biologique avec un composant de liaison. L'appareil comprend un corps définissant une poignée; au moins un tambour monté de manière rotative sur le corps; un levier d'actionnement du tambour monté sur le corps, le levier d'actionnement du tambour étant couplé au tambour pour faire tourner le tambour dans une direction prédéterminée lorsque le levier d'actionnement du tambour est déplacé; et un ensemble de sertissage couplé au corps pour maintenir un composant de sertissage et sertir sélectivement le composant de sertissage sur le composant de liaison. La poignée, le levier d'actionnement du tambour et l'ensemble de sertissage sont configurés, dimensionnés et positionnés de manière à ce que l'utilisateur puisse serrer le composant de liaison autour du tambour en déplaçant le levier d'actionnement du tambour d'une seule main; l'utilisateur étant capable d'actionner l'ensemble de sertissage pour sertir le composant de sertissage sur la section médiane du composant de liaison en utilisant une autre main tout en tenant simultanément la poignée avec la première main.

### Objet et résumé de l'invention

La présente invention a ainsi pour objet un ancillaire de mise en tension :
- permettant de mettre en tension un élément longiligne formant une boucle sur au moins une portion d'un corps vertébral sans endommager ledit corps vertébral, qui soit progressive et facilement ajustable par le chirurgien ;
- permettant d'exercer une tension égale sur chacune des deux extrémités de l'élément longiligne ;
- permettant au chirurgien de relâcher rapidement la tension exercée sur l'élément longiligne si besoin ;
- compact, facilitant ainsi son utilisation, et la disposition éventuelle de plusieurs ancillaires de mise en tension sur un même site opératoire ;
- de construction simplifiée afin qu'il soit à usage unique.

La présente invention a ainsi pour objet, selon un premier aspect, un ancillaire de mise en tension d'un élément longiligne pour la fixation d'un implant sur un élément osseux, notamment au moins une portion d'un corps vertébral, en entourant au moins partiellement ledit élément osseux, ledit ancillaire comprenant une tige ayant des extrémités proximale et distale et un axe longitudinal L, l'extrémité distale de ladite tige étant munie d'un dispositif d'appui pour prendre appui sur l'implant, ledit ancillaire comprend en outre un dispositif de mise en tension dudit élément longiligne. De manière avantageuse, le dispositif de mise en tension comprend un tambour monté rotatif autour d'un axe T, transverse à l'axe L, disposé au niveau de l'extrémité proximale de la tige et une portion de maintien configurée pour maintenir l'élément longiligne. Le dispositif de mise en tension comprend en outre un dispositif d'entraînement en rotation du tambour autour de l'axe T permettant la mise en tension de l'élément longiligne par l'enroulement de l'élément longiligne autour du tambour.

Avantageusement, l'élément longiligne adopte tout d'abord une trajectoire rectiligne selon l'axe longitudinale L de la tige puis est mis en tension par son enroulement autour du tambour. Le dispositif de mise en tension est donc très compact ce qui diminue son encombrement sur le site opératoire. Cette disposition est également avantageuse pour son stockage.

La mise en tension de l'élément longiligne est obtenu par enroulement de ce dernier autour du tambour qui est entraîné en rotation par le dispositif d'entraînement, la mise en tension est donc progressive.

Avantageusement, le pourtour du tambour forme une zone de stockage de l'élément longiligne à l'état enroulé au fur et à mesure de sa mise en tension. Cette disposition limite considérablement l'encombrement du dispositif de mise en tension comparativement aux ancillaires de mise en tension de l'état de la technique pour lesquels la mise en tension est obtenue par le déplacement en translation de l'élément mobile, en liaison avec l'élément longiligne, le long d'un axe.

Cette disposition permet également d'avoir plus de liberté dans le choix de la longueur de l'élément longiligne puisque la course de serrage, formée autour du tambour, est moins volumineuse que les courses de serrage dans l'état de la technique.

Avantageusement, l'agencement du dispositif de mise en tension selon l'extrémité proximale de la tige facilite sa manipulation et permet de l'écarter du champ opératoire.

On comprend, dans le présent texte, par « l'axe T est transverse à l'axe L » que ces deux axes sont sécants, en particulier l'axe T est sensiblement perpendiculaire à l'axe L.

De préférence, la distance séparant les extrémités distale et proximale de la tige est déterminée en sorte que le dispositif de mise en tension ne soit pas, en conditions d'utilisation, en contact avec le site de l'intervention chirurgicale. Seule l'extrémité distale, par l'intermédiaire du dispositif d'appui, et éventuellement une portion de la tige dans le prolongement de son extrémité distale, sont susceptibles de venir en contact avec le site de l'intervention lorsque le dispositif d'appui prend appui sur un implant.

De préférence, la distance séparant les extrémités distale et proximale de la tige est supérieure ou égale à 5 cm, encore de préférence supérieure ou égale à 10 cm.

De préférence, le dispositif pour prendre appui est configuré pour prendre appui sur un implant, que ce dernier soit une barre longitudinale implantable ou un implant permettant le maintien de l'élément longiligne en tension, ledit implant étant éventuellement configuré pour être solidarisé aussi à une barre longitudinale.

En fonctionnement, l'élément longiligne est placé en liaison avec la portion de maintien pour son maintien au tambour, puis le dispositif d'entrainement en rotation du tambour est actionné, en particulier manuellement, en sorte d'entrainer en rotation le tambour, et obtenir la mise en tension de l'élément longiligne par enroulement. Lorsque le dispositif d'entraînement n'est plus actionné, la mise en rotation et donc la mise en tension sont stoppées.

L'implant de maintien en tension de l'élément longiligne à l'état enroulé sur au moins une portion d'un corps vertébral peut être celui décrit dans la demande de brevet internationale PCT/FR2014/051801 déposée au nom de la demanderesse, et dont la description est incorporée dans le présent texte, ou encore avoir une configuration différente, notamment ne comprenant pas de moyens de solidarisation à une barre longitudinale implantable.

De préférence, le dispositif d'appui est moulé pour former un logement recevant par emboitement un implant de maintien en tension de l'élément longiligne à l'état enroulé sur au moins une portion d'un corps vertébral.

De préférence, le dispositif d'appui et/ou la tige et/ou au moins en partie le dispositif de mise en tension et/ou le tambour, et/ou au moins en partie le dispositif d'entraînement en rotation, sont dans un matériau polymérique, notamment renforcé, en particulier moulé.

La construction de l'ancillaire selon l'invention étant simplifiée, il est ainsi possible de fabriquer l'ancillaire par moulage d'une grande partie des pièces le constituant.

En particulier, le matériau polymérique comprend un ou plusieurs polymères, notamment en mélange avec des éléments de renforcement.

Le ou lesdits polymères peuvent être choisis parmi : le polyéthylène téréphtalate, le polycarbonate, le polyarylamide, le polyamide (tel que le polyamide 4-6, le polyamide 6-6, le polyamide 6, le polyamide 12 ou 11).

Les éléments de renforcement sont de préférence des fibres de renfort, encore de préférence en verre ou en carbone, notamment en verre.

Les fibres de carbone comprennent notamment une proportion massique en carbone supérieure ou égale à 90%.

Le matériau polymérique peut ainsi être un polyarylamide chargé en fibres de verre, tel que celui commercialisé sous la marque IXEF^{®} par Solvay.

De préférence, l'ancillaire est à usage unique. Cette disposition est facilitée du fait du moulage, de la totalité ou d'une partie des pièces formant l'ancillaire.

Cette disposition limite les risques de propagation des germes, et donc d'infection des patients, en particulier par les maladies nosocomiales.

En effet, les ancillaires de mise en tension dans l'état de la technique sont stérilisés en vue de leur réutilisation, or cette stérilisation représente non seulement un surcout mais s'avère délicate compte tenu de leur construction mettant en jeu de nombreuses pièces.

Avantageusement, l'ancillaire de mise en tension selon l'invention, lorsqu'il est à usage unique, évite une opération de stérilisation en vue de sa réutilisation, limitant ainsi les risques infectieux.

La portion de maintien du tambour est configurée pour maintenir une, ou deux, extrémité(s) libre(s) de l'élément longiligne.

De préférence, l'élément longiligne est un élément textile, qui peut être tressé, tricoté ou tissé, de préférence tressé. Encore de préférence, l'élément longiligne est plat afin de générer des forces de frottement importantes avec les surfaces contre lesquelles il se déplace, améliorant ainsi les forces de serrage. Cette disposition limite également l'abrasion des surfaces en contact avec l'élément longiligne en comparaison avec un élément longiligne mince et sensiblement cylindrique.

L'invention est déclinée ci-après dans une série de variantes de réalisation, qui peuvent être considérées seules ou en combinaison avec une ou plusieurs des précédentes.

Le dispositif d'entrainement en rotation comprend un levier monté pivotant autour de l'axe T, et un dispositif d'embrayage configuré pour que le pivotement du levier selon le sens de rotation R1 dans une première position entraîne en rotation le tambour autour de l'axe T.

Cette disposition facilite la préhension du dispositif d'entraînement, et améliore son ergonomie.

De préférence, le levier est une poignée ayant une forme convergeant vers sa partie avant.

De plus, le dispositif d'entraînement comprend un dispositif de débrayage configuré, pour qu'en position débrayée, le levier étant pivotée autour de l'axe T selon un sens de rotation R2 opposé au sens de rotation R1, le tambour soit libre de tourner autour de l'axe T.

Cette disposition permet de libérer la tension exercée sur l'élément longiligne.

Cette opération peut être effectuée en fin de serrage pour pouvoir couper l'élément longiligne au plus près de l'implant de maintien en tension de l'élément longiligne à l'état enroulé sur au moins une portion d'un corps vertébral, disposé en fonctionnement au niveau du dispositif d'appui.

Dans une variante de réalisation, le dispositif d'entrainement comprend un dispositif de rappel configuré pour qu'une fois le levier libéré de la pression exercée sur ce dernier pour son pivotement dans le sens de rotation R1 dans la première position, le levier pivote dans un sens de rotation R2, opposé au sens de rotation R1, dans une position au repos.

Cette disposition permet d'effectuer un serrage par paliers. L'actionnement du levier enroule d'une longueur déterminée l'élément longiligne autour du tambour, permettant ainsi son serrage. Il est donc nécessaire d'actionner plusieurs fois le levier jusqu'à obtenir la mise en tension désirée.

Dans une variante de réalisation, le dispositif d'entraînement comprend un dispositif anti-retour configuré pour empêcher la rotation du tambour dans un sens de rotation R2, opposé au sens de rotation R1, dans la première position, et éventuellement dans la position au repos.

Dans une variante de réalisation, le dispositif d'entrainement comprend une pince de préhension comprenant des première et seconde portions, la première portion formant ledit levier et la seconde portion étant fixe avec la tige.

De préférence, la première portion et ladite seconde portion comprennent chacune des parties proximales et distales, lesdites parties proximales ayant chacune une forme convergente dans une direction opposée à leurs parties distales, notamment en forme de poignée.

De préférence, la seconde portion, ayant des parties proximale et distale, est dans le prolongement de la tige, encore de préférence, le dispositif d'appui pour prendre appui et/ou la tige et/ou la seconde portion est/sont moulé(e)(s) en une seule pièce, en particulier dans un matériau polymérique (renforcé ou non) tel que défini ci-dessus.

De préférence, la partie distale de la seconde portion a une forme de boitier (décrit également ci-après) configuré pour recevoir en rotation le tambour.

Dans une variante de réalisation, le rapprochement des première et seconde portions de la pince de préhension, par pivotement de la première portion formant le levier vers la seconde portion, entraine la mise en rotation du tambour.

Cette disposition améliore encore l'ergonomie de l'ancillaire, en facilitant l'actionnement manuelle du dispositif d'entraînement.

Dans une variante de réalisation, le dispositif d'embrayage est un mécanisme à cliquet, en particulier comprenant une première roue dentée, et éventuellement une seconde roue dentée, en liaison en rotation autour de l'axe T avec le tambour, et une pièce à cliquet en liaison avec le levier.

En particulier, la pièce à cliquet est en liaison mobile avec le levier.

Avantageusement, la pièce à cliquet est configurée pour coopérer avec la première roue dentée, et éventuellement avec la seconde roue dentée, en sorte que le pivotement du levier selon le sens de rotation R1 dans une première position engrène la pièce à cliquet, en liaison avec le levier, avec la première roue dentée, et éventuellement la seconde roue dentée, faisant ainsi tourner la ou les roue(s) dentée(s) et le tambour.

De préférence, la liaison entre la pièce à cliquet et le levier est configurée en sorte que la pièce à cliquet ait une position haute, dans laquelle la pièce à cliquet est désengagée de la ou des roue(s) dentée(s), et une position basse, dans laquelle la pièce à cliquet coopère avec les dents de la ou des roue(s) dentée(s).

En particulier, la pièce à cliquet en liaison avec le levier est dans une position basse lorsque le levier est pivoté dans le sens de rotation R1 dans la première position, et éventuellement lorsque le levier est dans la position au repos.

En particulier, la pièce à cliquet est dans une position haute, lorsque le levier est en position débrayée.

En particulier, la pièce à cliquet en liaison avec le levier alterne les positions haute et basse, d'une part, lors de son passage de la première position à la position au repos, et lors de son passage de la position au repos à la position débrayée.

De préférence, la pièce à cliquet est en liaison avec le levier par l'intermédiaire d'un plot fixé à ladite pièce à cliquet et monté coulissant dans une glissière fixée au levier, encore de préférence un ressort est disposé autour du plot.

Dans une variante de réalisation, le dispositif anti-retour comprend une pièce à cliquet, notamment en liaison avec la seconde portion de la pince, configurée pour coopérer avec la première roue dentée, et éventuellement la seconde roue dentée.

De préférence, la liaison entre la pièce à cliquet et la seconde portion est configurée en sorte que la pièce à cliquet ait une position haute, dans laquelle la pièce à cliquet est désengagée de la ou des roue(s) dentée(s), et une position basse, dans laquelle la pièce à cliquet coopère avec la ou les roue(s) dentée(s).

En particulier, la pièce à cliquet en liaison avec la seconde portion est dans une position basse lorsque le levier est dans la première position, et éventuellement dans la position au repos.

En particulier, la pièce à cliquet en liaison avec la seconde portion est dans une position haute, lorsque le levier est en position débrayée.

En particulier, la pièce à cliquet, en liaison avec la seconde portion, alterne les positions haute et basse, lors du pivotement du levier selon le sens de rotation R1 dans la première position.

De préférence, la pièce à cliquet est en liaison avec la seconde portion par l'intermédiaire d'un plot fixé à ladite pièce à cliquet et monté coulissant dans une glissière fixée à la seconde portion, encore de préférence un ressort est disposé autour du plot.

Dans une variante, le dispositif de mise en tension comprend un boitier recevant le tambour, ledit boitier étant en liaison fixe avec l'extrémité proximale de la tige (en particulier ledit boitier forme la partie distale de la seconde portion de la pince).

Dans une variante, le tambour comprend deux extrémité latérales, et le boitier comprend des joues droite et gauche ayant chacune une ouverture recevant une extrémité latérale du tambour.

Dans une variante, le levier comprend une partie proximale (configurée pour être préhensible) et une partie distale, ladite partie distale comprend deux projections latérales entre lesquelles est disposé le boitier, et éventuellement les première et seconde roues dentées disposées de part et d'autre du boitier.

Dans une variante, le dispositif de débrayage comprend au moins une came, de préférence deux cames, en particulier supportée par le boitier, configurée(s) pour désactiver le mécanisme cliquet, en particulier désengager la pièce à cliquet en liaison avec le levier de la première roue dentée, et éventuellement de la seconde roue dentée.

Dans une variante, le dispositif de débrayage comprend au moins une came, de préférence au moins deux cames, en particulier supportée par le levier, plus particulièrement par la partie distale de la première portion formant le levier, configurée pour désengager la pièce à cliquet en liaison avec la seconde portion de la première roue dentée, et éventuellement de la seconde roue dentée.

Les deux variantes précitées, considérées seules ou en combinaison, permettent de placer le levier en position débrayée.

Dans une variante de réalisation, le levier (notamment sa partie distale) et/ou le boitier comprennent chacun deux fentes traversantes recevant chacun une extrémité latérale d'une pièce à cliquet, avec laquelle il(s) est(sont) en liaison. Lesdites fentes sont configurées, de préférence, pour que les extrémités latérales de la ou des pièce(s) à cliquet translatent dans ces dernières, notamment d'une position haute à une position basse.

Dans une variante, la portion de maintien est une fente traversante du tambour permettant le passage d'au moins une extrémité libre, de préférence des deux extrémités libres, de l'élément longiligne.

Avantageusement, il n'est pas nécessaire de solidariser l'élément longiligne par l'utilisation de moyens de solidarisation complexes et fastidieux à mettre en œuvre. Le chirurgien place ainsi au moins une extrémité libre de l'élément longiligne à travers la fente du tambour, puis l'enroulement de l'élément longiligne sur lui-même lors de la mise en rotation du tambour permet le blocage de l'élément longiligne sur lui-même et sa solidarisation au tambour.

La présente invention a pour objet, selon un deuxième aspect, un ensemble comprenant un ancillaire de mise en tension selon l'une quelconque des variantes de réalisation précédentes selon un premier aspect, et au moins un implant, en particulier choisi parmi une barre longitudinale implantable ou un implant de maintien en tension de l'élément longiligne à l'état enroulé sur au moins une portion d'un corps vertébral.

La présente invention sera mieux comprise à la lecture d'un exemple de réalisation décrit ci-après, cité à titre non limitatif et illustré par les dessins décrits ci-après annexés au présent texte.

### Description détaillée des dessins

- la figure **1** est une représentation schématique et en perspective d'un exemple d'implant de maintien en tension de l'élément longiligne à l'état enroulé sur au moins une portion d'un corps vertébral comprenant également des moyens de solidarisation à une barre longitudinale implantable ;
- la figure **2** est une représentation schématique et en perspective d'un exemple d'ancillaire de mise en tension selon l'invention ;
- la figure **3** est une représentation schématique et en perspective d'une vue éclatée de l'ancillaire de mise en tension représenté à la figure **2** ;
- la figure **4** est une représentation schématique, vu de dessus, du levier du dispositif de mise en tension selon l'invention de l'ancillaire représenté sur les figures **2** et **3** **;**
- la figure **5** est une représentation schématique de l'ancillaire dont le dispositif de mise en tension est représenté selon le plan de coupe V-V à la figure **4** ;
- la figure **6** est une représentation schématique et en perspective d'un ensemble selon la présente invention comprenant l'ancillaire de mise en tension représenté aux figures 2 à 5, en fonctionnement, et l'implant de maintien en tension de l'élément longiligne à l'état enroulé sur au moins une portion d'un corps vertébral comprenant également des moyens de solidarisation à une barre longitudinale implantable.

### Description détaillée des exemples de réalisation

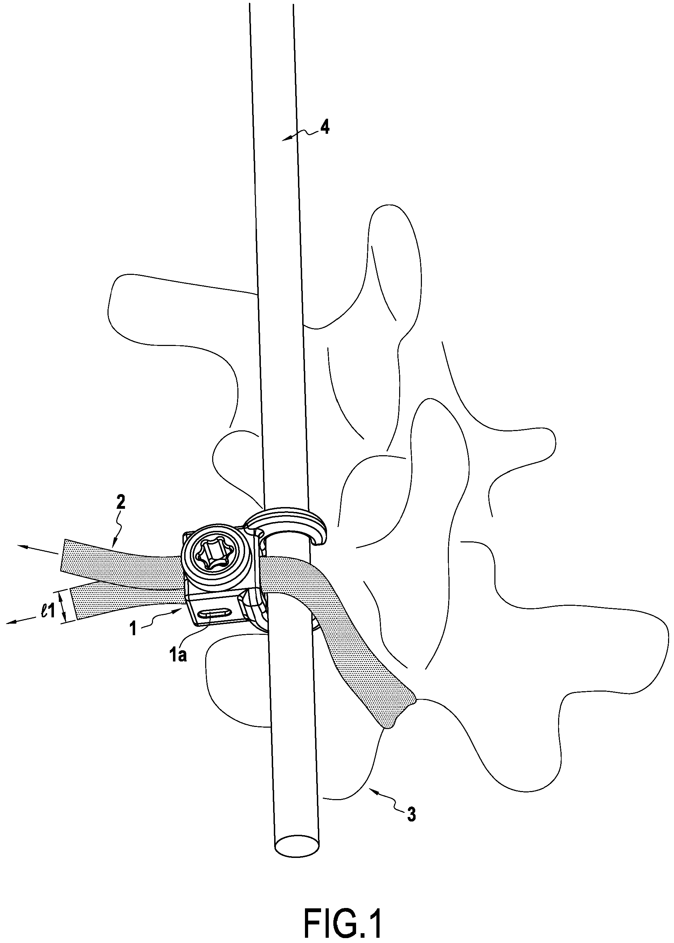

La figure **1** représente un implant de maintien en tension **1** de l'élément longiligne **2** à l'état enroulé sur au moins une portion d'un corps vertébral **3,** ledit implant **1** comprenant, dans cet exemple précis, également des moyens de solidarisation **5,6** à une barre longitudinale implantable **4.** Ledit élément longiligne **2** plat, ayant une largeur **l1,** préalablement à sa fixation dans son état enroulé autour de la portion du corps vertébral **3** a été mis en tension à l'aide d'un ancillaire de mise en tension selon l'invention.

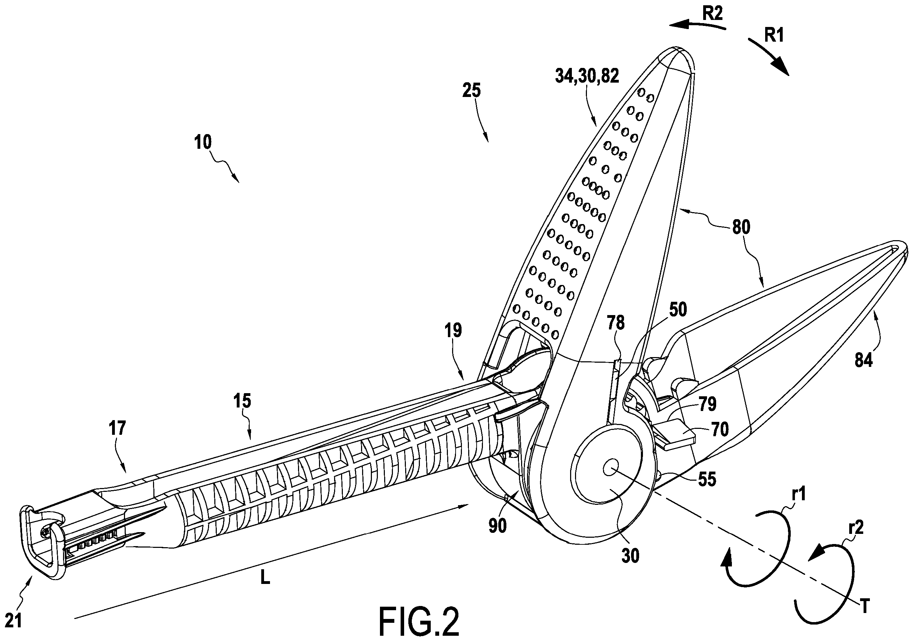

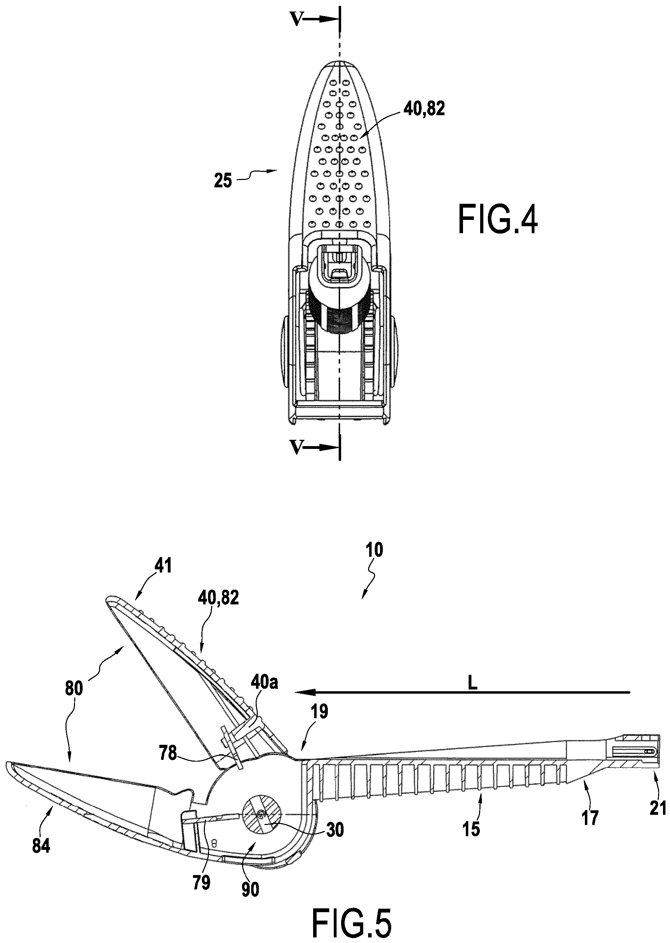

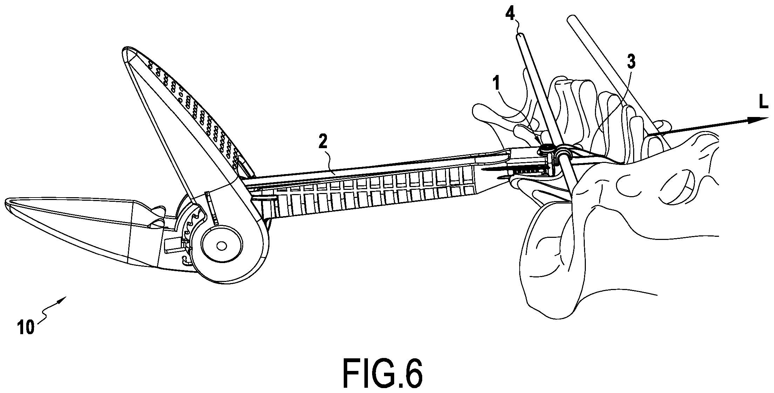

L'exemple d'ancillaire de mise en tension **10** selon l'invention, représenté aux figures 2 à 6, comprend une tige **15** ayant des extrémités distale **17** et proximale **19** et un axe longitudinal **L,** l'extrémité distale **17** de ladite tige **15** étant munie d'un dispositif d'appui **21** pour prendre appui sur l'implant **1.** L'ancillaire **10** comprend en outre un dispositif de mise en tension **25** dudit élément longiligne **2.** Le dispositif de mise en tension **25** comprend un tambour **30** monté rotatif autour de l'axe **T,** transverse à l'axe **L,** en particulier sensiblement perpendiculaire à ce dernier, disposé au niveau de l'extrémité proximale **19** de la tige **15** et comprend une portion de maintien **32** adaptée pour coopérer avec l'élément longiligne **2** pour son maintien. Le dispositif de mise en tension **25** comprend en outre un dispositif d'entraînement en rotation **34** du tambour **30** autour de l'axe T permettant la mise en tension de l'élément longiligne 2 par l'enroulement de l'élément longiligne **2** autour du tambour **30.**

Le dispositif d'entrainement **34** comprend un levier **40** monté pivotant autour de l'axe **T,** et un dispositif d'embrayage **42** configuré pour que le pivotement du levier **40** selon le sens de rotation R1 dans une première position entraîne en rotation le tambour **30** autour de l'axe **T.**

Le dispositif d'embrayage **42** est un mécanisme à cliquet **45** comprenant une première roue dentée **46** et une seconde roue dentée **48,** chacune étant en liaison en rotation autour de l'axe T avec le tambour **30,** ainsi qu'une pièce à cliquet **50** en liaison, en particulier mobile, avec le levier **40.** La pièce à cliquet **50** est en liaison avec le levier **40** par l'intermédiaire d'un plot **51** fixé à ladite pièce à cliquet **50** et monté coulissant dans une glissière **400** fixée au levier **40,** encore de préférence un ressort **52** est disposé autour du plot **51.**

Le dispositif d'entraînement **34** comprend également un dispositif de débrayage **55** configuré, pour qu'en position débrayée, le levier **40** étant pivotée autour de l'axe T selon un sens de rotation R2 opposé au sens de rotation R1, le tambour **30** soit libre de tourner autour de l'axe **T.**

Le dispositif d'entrainement **34** comprend un dispositif de rappel **58** configuré pour qu'une fois le levier **40** libéré de la pression exercée sur ce dernier pour son pivotement dans le sens de rotation R1 dans la première position, le levier **40** pivote dans un sens de rotation R2, opposé au sens de rotation R1, dans une position au repos.

Le dispositif d'entraînement **34** comprend un dispositif anti-retour **70** configuré pour empêcher la rotation du tambour **30** dans un sens de rotation R2, opposé au sens de rotation R1, lorsque le levier **40** est dans la première position, et dans la position au repos.

Le dispositif d'entrainement **34** comprend également une pince de préhension **80** comprenant des première **82** et seconde **84** portions, la première portion **82** étant ledit levier **40** et la seconde portion **84** étant fixe avec la tige **15.**

La seconde portion **84** comprend une partie proximale **85** et une partie distale **86.** La partie distale **86** forme le boitier **90.** Les parties proximales **85** et **41,** respectivement des première **82** et seconde **84** portions, sont convergentes et forment chacune une poignée de préhension.

Le dispositif anti-retour **70** comprend une pièce à cliquet **71,** notamment en liaison avec la seconde portion **84** de la pince **80,** configurée pour coopérer avec la première roue dentée **46,** et la seconde roue dentée **48.** La pièce à cliquet **71** est en liaison, en particulier mobile, avec la seconde portion **84** par l'intermédiaire d'un plot **72** fixé à ladite pièce à cliquet **71** et monté coulissant dans une glissière (non représentée) fixée à la seconde portion **84,** de préférence un ressort **73** est disposé autour du plot **72.**

Le dispositif de mise en tension **25** comprend un boitier **90** recevant le tambour **30,** ledit boitier **90** étant en liaison fixe avec l'extrémité proximale **19** de la tige **15.**

Le levier **40** comprend une partie proximale **41** et une partie distale **43,** ladite partie distale **43** comprend deux projections latérales **44,49** entre lesquelles est disposé le boitier **90,** et les première **46** et seconde **48** roues dentées disposées de part et d'autre du boitier **90.**

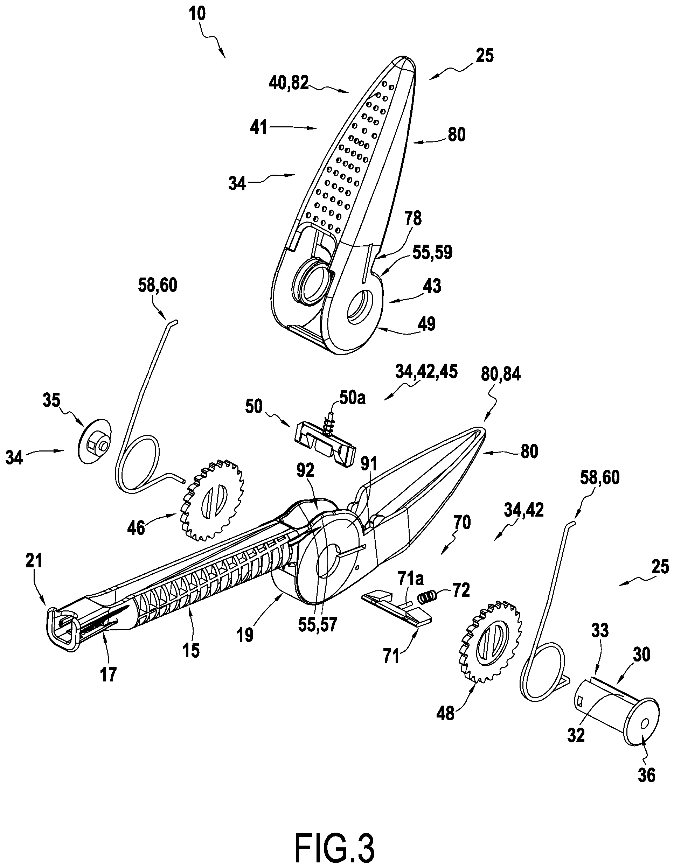

Le tambour **30** comprend deux extrémités latérales **31,33,** et le boitier **90** comprend des joues droite **91** et gauche **92** ayant chacune une ouverture, seule l'ouverture **93** est visible sur la figure 3, recevant une extrémité latérale **31,33** du tambour **30.**

Le tambour **30** comprend également deux caches de fermeture latérales **35,36** solidarisant en rotation le tambour **30** au levier **40** et à la seconde portion **84.**

Le dispositif de débrayage **55** comprend deux cames **57** montées sur le boitier **90,** en particulier des profils extérieurs de came **57,** configurées pour désactiver le mécanisme à cliquet **45,** en particulier désengager la pièce à cliquet **50** en liaison avec le levier **40** de la première roue dentée **46,** et de la seconde roue dentée **48.**

Le dispositif de débrayage **55** comprend en outre deux cames **59** montées sur le levier **40,** en particulier des profils extérieurs de came **59,** plus particulièrement montées sur la partie distale **43** de la première portion **82** formant le levier **40,** configurées pour désengager la pièce à cliquet **71,** en liaison avec la seconde portion **84,** de la première roue dentée **46,** et de la seconde roue dentée **48.**

Le dispositif de débrayage **55** comprend également deux paires de fentes **78,79** recevant les extrémités latérales des pièces à cliquet **50** et 70.

Dans une variante, la portion de maintien **32** est une fente traversante du tambour **30** permettant le passage d'au moins une extrémité libre, de préférence des deux extrémités libres, de l'élément longiligne **2.**

Dans cet exemple précis, le dispositif de rappel **58** comprend deux ressorts de rappel **60** montés en rotation autour de l'axe T. Une portion de chacun des ressorts est montée sur le tambour **30** et une autre portion longitudinale de chacun des ressorts **60** est configurée pour venir en appui et exercer une force de rappel sur le levier **40** dans le sens de rotation R2.

En fonctionnement, au moins une extrémité de l'élément longiligne **2,** de préférence les deux extrémités libres, en provenance de l'implant de maintien en tension **1,** est/sont passée(s) dans la portion de maintien **32,** en particulier au travers de la fente traversante, puis l'utilisateur rapproche les première **82** et seconde **84** portions de la pince de préhension **80,** par pivotement de la première portion **82** formant le levier **40** vers la seconde portion **84.** Ce pivotement entraine la mise en rotation du tambour **30,** et donc l'enroulement de l'élément longiligne **2** sur lui-même, lequel s'auto-bloque et est donc maintenu à la portion de maintien **32.** La première portion **82** de la pince **80** est pivotée jusqu'à ce qu'elle soit en contact avec la seconde portion **84,** dans la première position. Lors du pivotement de la première portion **82** correspondant au levier **40,** le levier **40** passe de sa position au repos, dans laquelle la pièce à cliquet **50** et la pièce à cliquet **71** sont en position basse, c'est-à-dire engrènent les première **46** et seconde **48** roues dentées, à la première position, dans laquelle, la pièce à cliquet **50** reste en position basse et la pièce à cliquet **71** passe de la position basse à la position haute en fonction de l'avancée des roues dentées **46** et **48** assurant ainsi la fonction de disposition anti-retour **70.** Lorsque l'utilisateur relâche le levier **40,** ce dernier sous l'effet du dispositif de rappel **58,** revient dans sa position au repos, la pièce à cliquet **50** adopte alors des positions haute et basse tandis que la pièce à cliquet **71** reste en position basse conservant ainsi l'enroulement de l'élément longiligne **2** effectué autour du tambour **30.** L'utilisateur pivote le levier **40** de la pince **80** vers sa seconde portion **84,** passant de la position au repos à la première position, autant de fois que nécessaire jusqu'à obtenir l'enroulement désiré de l'élément longiligne **2** autour du tambour **30** et donc la mise en tension appropriée de l'élément longiligne **2** autour de ladite au moins une portion du corps vertébrale. L'utilisateur verrouille alors l'implant de maintien en tension **1** afin de maintenir à l'état serré l'élément longiligne **2** sur au moins une barre longitudinale et au moins une portion de corps vertébral.

L'utilisateur pivote alors le levier **40** selon le sens de rotation R2, à l'opposé du sens de rotation R1, jusqu'à venir en butée contre la tige **15,** dans une position débrayée. Le dispositif de débrayage **55,** par l'intermédiaire notamment du jeu de cames **57** et **59,** désengrènent les pièces à cliquet **50** et **71** des roues dentées **46** et **48** de sorte que le tambour **30** est libre en rotation. L'utilisateur peut alors couper l'élément longiligne **2** au niveau de l'implant **1** afin de supprimer la longueur d'élément longiligne **2** non nécessaire au serrage.

De préférence, toutes les pièces de l'ancillaire de mise en tension **10** sont dans un matériau polymérique moulé, en particulier renforcé à l'aide de fibres de renfort. L'ancillaire de mise en tension est ainsi de préférence à usage unique de sorte qu'une fois la mise en tension effectuée, l'ancillaire de mise en tension **10** est mis au rebus. Il est également possible de recycler le matériau polymérique.

Dans cet exemple précis, le dispositif d'appui **21** comprend un logement configuré pour recevoir par enclipsage l'implant **1.** Le dispositif d'appui selon l'invention n'est pas limité pour prendre appui sur ce type d'implant **1,** et peut être configuré afin de coopérer pour prendre appui sur d'autres types d'implants implant de maintien en tension **1** de l'élément longiligne **2** à l'état enroulé sur au moins une portion d'un corps vertébral, et/ou encore pour prendre directement appui sur une barre longitudinale implantable.

Avantageusement, le dispositif d'appui selon l'invention peut-être moulé selon la configuration de l'implant de maintien en tension de l'élément longiligne sélectionné.

## Revendications

1. Ancillaire de mise en tension (10) d'un élément longiligne (2) pour la fixation d'un implant (1,4) sur un élément osseux, notamment au moins une portion d'un corps vertébral (3), en entourant au moins partiellement ledit élément osseux (3), ledit ancillaire (10) comprenant une tige (15) ayant des extrémités proximale (19) et distale (17) et un axe longitudinal L, l'extrémité distale (17) de ladite tige (15) étant munie d'un dispositif d'appui (21) pour prendre appui sur l'implant (1), ledit ancillaire (10) comprend en outre un dispositif de mise en tension (25) dudit élément longiligne (2), **caractérisé en ce que** ledit dispositif de mise en tension (25) comprend un tambour (30) monté rotatif autour d'un axe T, transverse à l'axe L, disposé au niveau de l'extrémité proximale (19) de la tige (15) et comprenant une portion de maintien (32) adaptée pour coopérer avec l'élément longiligne (2), et **en ce que** ledit dispositif de mise en tension (25) comprend en outre un dispositif d'entraînement en rotation (34) du tambour (30) autour de l'axe T permettant la mise en tension de l'élément longiligne (2) par l'enroulement de l'élément longiligne (2) autour du tambour (30) **en ce que** le dispositif d'entrainement (34) comprend un levier (40) monté pivotant autour de l'axe T, et un dispositif d'embrayage (42) configuré pour que le pivotement du levier (40) selon le sens de rotation R1 dans une première position entraîne en rotation le tambour (30) autour de l'axe T, et **en ce que** le dispositif d'entraînement (34) comprend un dispositif de débrayage (55) configuré, pour qu'en position débrayée, le levier (40) étant pivotée autour de l'axe T selon un sens de rotation R2 opposé au sens de rotation R1, le tambour (30) soit libre de tourner autour de l'axe T .

2. Ancillaire de mise en tension (10) selon la revendication 1, **caractérisé en ce que** le dispositif d'entrainement (34) comprend un dispositif de rappel (58) configuré pour qu'une fois le levier (40) libéré de la pression exercée sur ce dernier pour son pivotement dans le sens de rotation R1 dans la première position, le levier (40) pivote dans un sens de rotation R2, opposé au sens de rotation R1, dans une position au repos.

3. Ancillaire de mise en tension (10) selon l'une ou l'autre des revendications 1 et 2, **caractérisé en ce que** le dispositif d'entraînement (34) comprend un dispositif anti-retour (70) configuré pour empêcher la rotation du tambour (30) dans un sens de rotation R2, opposé au sens de rotation R1, lorsque le levier (40) est dans la première position, et éventuellement dans la position au repos.

4. Ancillaire de mise en tension (10) selon l'une quelconque des revendications 1 à 3, **caractérisé en ce que** le dispositif d'entrainement (34) comprend une pince de préhension (80) comprenant des première (82) et seconde (84) portions, la première portion (82) étant ledit levier (40) et la seconde portion (84) étant fixe avec la tige (15).

5. Ancillaire de mise en tension (10), selon la revendication 4, **caractérisé en ce que** le rapprochement des première (82) et seconde (84) portions de la pince de préhension (80), par pivotement de la première portion (82) formant le levier (40) vers la seconde portion (84), entraine la mise en rotation du tambour (30).

6. Ancillaire de mise en tension (10) selon l'une quelconque des revendications 1 à 5, **caractérisé en ce que** le dispositif d'embrayage (42) est un mécanisme à cliquet (45), en particulier comprenant une première roue dentée (46), et éventuellement une seconde roue dentée (48), en liaison en rotation autour de l'axe T avec le tambour (30), et une pièce à cliquet (50) en liaison avec le levier (40).

7. Ancillaire de mise en tension (10) selon la revendication 6, **caractérisé en ce que** le dispositif anti-retour (70) comprend une pièce à cliquet (71), notamment en liaison avec la seconde portion (84) de la pince (80), configurée pour coopérer avec la première roue dentée (46), et éventuellement la seconde roue dentée (48).

8. Ancillaire de mise en tension (10) selon l'une quelconque des revendications **1** à 7, **caractérisé en ce que** le dispositif de mise en tension (25) comprend un boitier (90) recevant le tambour (30), ledit boitier (90) étant en liaison fixe avec l'extrémité proximale (19) de la tige (15).

9. Ancillaire de mise en tension (10) selon la revendication 8, **caractérisé en ce que** le tambour (30) comprend deux extrémité latérales (31,33), et **en ce que** ledit boitier (90) comprend des joues droite (91) et gauche (92) ayant chacune une ouverture (93) recevant une extrémité latérale (31,33) du tambour (30).

10. Ancillaire de mise en tension (10) selon la revendication 9, **caractérisé en ce que** le levier (40) comprend une partie proximale (41) et une partie distale (43), ladite partie distale (43) comprend deux projections latérales (44,49) entre lesquelles est disposé le boitier (90), et éventuellement les première (46) et seconde (48) roues dentées disposées de part et d'autre du boitier (90).

11. Ancillaire de mise en tension (10) selon l'une quelconque des revendications 1, et 6 à 10, **caractérisé en ce que** le dispositif de débrayage (55) comprend au moins une came (57,59), en particulier montée sur le boitier (90), configurée pour désactiver le mécanisme à cliquet (45), en particulier désengager la pièce à cliquet (50) en liaison avec le levier (40) de la première roue dentée (46), et éventuellement de la seconde roue dentée (48).

12. Ancillaire de mise en tension (10) selon l'une quelconque des revendications **1** à 11, **caractérisé en ce que** la portion de maintien (32) est une fente traversante du tambour (30) permettant le passage d'au moins une extrémité libre, de préférence des deux extrémités libres, de l'élément longiligne (2).

13. Ensemble **caractérisé en ce qu'**il comprend un ancillaire de mise en tension (10) selon l'une quelconque des revendications **1** à 12, et au moins un implant (1,4), en particulier choisi parmi une barre longitudinale implantable (4) ou un implant de maintien en tension (1) de l'élément longiligne à l'état enroulé sur au moins une portion d'un corps vertébral.

## Patentansprüche

1. Spannhilfsmittel (10) eines länglichen Elements (2) zur Befestigung eines Implantats (1,4) an einem Knochenelement, insbesondere zumindest einem Abschnitt eines Wirbelkörpers (3), der das Knochenelement (3) zumindest teilweise umgibt, wobei das Hilfsmittel (10) einen Stab (15) mit proximalen (19) und distalen (17) Enden und einer Längsachse L umfasst, wobei das distale Ende (17) des Stabs (15) mit einer Stützvorrichtung (21) zur Stütze auf dem Implantat (1) versehen ist, wobei das Hilfsmittel (10) ferner eine Spannvorrichtung (25) des länglichen Elements (2) umfasst, **dadurch gekennzeichnet, dass** die Spannvorrichtung (25) eine Trommel (30) umfasst, die um eine Achse T quer zu der Achse L drehgelagert ist, auf Höhe des proximalen Endes (19) des Stabs (15) angeordnet ist und einen Halteabschnitt (32) umfasst, der dazu ausgelegt ist, mit dem länglichen Element (2) zusammenzuwirken, und dass die Spannvorrichtung (25) ferner eine Drehantriebsvorrichtung (34) der Trommel (30) um die Achse T umfasst, die das Spannen des länglichen Elements (2) durch Aufwickeln des länglichen Elements (2) um die Trommel (30) ermöglicht, dass die Antriebsvorrichtung (34) einen Hebel (40), der schwenkbar um die Achse T gelagert ist, und eine Kupplungsvorrichtung (42), die dazu ausgestaltet ist, dass das Schwenken des Hebels (40) entlang der Drehrichtung R1 in eine erste Position eine Drehung der Trommel (30) um die Achse T antreibt, umfasst, und dass die Antriebsvorrichtung (34) eine Auskupplungsvorrichtung (55) umfasst, die dazu ausgestaltet ist, dass in ausgekuppelter Position der Hebel (40) um die Achse T entlang einer Drehrichtung R2 entgegengesetzt zu der Drehrichtung RI geschwenkt wird, wobei sich die Trommel (30) frei um die Achse T drehen kann.

2. Spannhilfsmittel (10) nach Anspruch 1, **dadurch gekennzeichnet, dass** die Antriebsvorrichtung (34) eine Rückstellvorrichtung (58) umfasst, die dazu ausgestaltet ist, dass, sobald der Hebel (40) von dem Druck befreit ist, der für seine Schwenkung in der Drehrichtung R1 in der ersten Position auf ihn ausgeübt wird, der Hebel (40) in einer Drehrichtung R2 entgegengesetzt zu der Drehrichtung R1 in eine Ruheposition schwenkt.

3. Spannhilfsmittel (10) nach einem der Ansprüche 1 und 2, **dadurch gekennzeichnet, dass** die Antriebsvorrichtung (34) eine Rücklaufsperrvorrichtung (70) umfasst, die dazu ausgestaltet ist, die Drehung der Trommel (30) in einer Drehrichtung R2 entgegengesetzt zu der Drehrichtung R1 zu verhindern, wenn der Hebel (40) in der ersten Position und optional in der Ruheposition ist.

4. Spannhilfsmittel (10) nach einem der Ansprüche 1 bis 3, **dadurch gekennzeichnet, dass** die Antriebsvorrichtung (34) eine Greifzange (80) umfasst, die erste (82) und zweite (84) Abschnitte umfasst, wobei der erste Abschnitt (82) der Hebel (40) ist und der zweite Abschnitt (84) fest mit dem Stab (15) ist.

5. Spannhilfsmittel (10) nach Anspruch 4, **dadurch gekennzeichnet, dass** das Zusammenbringen des ersten (82) und des zweiten (84) Abschnittes der Greifzange (80) durch Schwenken des ersten Abschnittes (82), der den Hebel (40) bildet, zu dem zweiten Abschnitt (84) die Drehung der Trommel (30) antreibt.

6. Spannhilfsmittel (10) nach einem der Ansprüche 1 bis 5, **dadurch gekennzeichnet, dass** die Kupplungsvorrichtung (42) ein Ratschenmechanismus (45) ist, insbesondere umfassend ein erstes Zahnrad (46) und optional ein zweites Zahnrad (48) in Drehverbindung um die Achse T mit der Trommel (30), und ein Ratschenteil (50) in Verbindung mit dem Hebel (40).

7. Spannhilfsmittel (10) nach Anspruch 6, **dadurch gekennzeichnet, dass** die Rücklaufsperrvorrichtung (70) ein Ratschenteil (71) umfasst, insbesondere in Verbindung mit dem zweiten Abschnitt (84) der Zange (80), das dazu ausgestaltet ist, mit dem ersten Zahnrad (46) und optional dem zweiten Zahnrad (48) zusammenzuwirken.

8. Spannhilfsmittel (10) nach einem der Ansprüche 1 bis 7, **dadurch gekennzeichnet, dass** die Spannvorrichtung (25) ein Gehäuse (90) umfasst, das die Trommel (30) aufnimmt, wobei das Gehäuse (90) in fester Verbindung mit dem proximalen Ende (19) des Stabs (15) ist.

9. Spannhilfsmittel (10) nach Anspruch 8, **dadurch gekennzeichnet, dass** die Trommel (30) zwei seitliche Enden (31, 33) umfasst und dass das Gehäuse (90) rechte (91) und linke Wangen (92) umfasst, die jeweils eine Öffnung (93) aufweisen, die ein seitliches Ende (31, 33) der Trommel (30) aufnimmt.

10. Spannhilfsmittel (10) nach Anspruch 9, **dadurch gekennzeichnet, dass** der Hebel (40) einen proximalen Teil (41) und einen distalen Teil (43) umfasst, wobei der distale Teil (43) zwei seitliche Vorsprünge (44, 49) umfasst, zwischen denen das Gehäuse (90) angeordnet ist, und optional das erste (46) und das zweite (48) Zahnrad auf beiden Seiten des Gehäuses (90) angeordnet sind.

11. Spannhilfsmittel (10) nach einem der Ansprüche 1 und 6 bis 10, **dadurch gekennzeichnet, dass** die Auskupplungsvorrichtung (55) zumindest einen Nocken (57, 59) umfasst, der insbesondere auf dem Gehäuse (90) gelagert ist, der dazu ausgestaltet ist, den Ratschenmechanismus (45) zu deaktivieren, insbesondere das Ratschenteil (50) in Verbindung mit dem Hebel (40) des ersten Zahnrads (46) und optional des zweiten Zahnrads (48) zu lösen.

12. Spannhilfsmittel (10) nach einem der Ansprüche 1 bis 11, **dadurch gekennzeichnet, dass** der Halteabschnitt (32) ein Durchgangsschlitz der Trommel (30) ist, der den Durchgang von zumindest einem freien Ende, bevorzugt der zwei freien Enden, des länglichen Elements (2) ermöglicht.

13. Anordnung, **dadurch gekennzeichnet, dass** sie ein Spannhilfsmittel (10) nach einem der Ansprüche 1 bis 12 und zumindest ein Implantat (1, 4), insbesondere ausgewählt aus einer implantierbaren Längsstange (4) oder einem Implantat zur Spannungserhaltung (1) des länglichen Elements in dem aufgewickelten Zustand an zumindest einem Abschnitt eines Wirbelkörpers umfasst.

## Claims

1. An ancillary for tensioning (10) an elongate element (2) for the attachment of an implant (1, 4) on a bone element, in particular at least one portion of a vertebral body (3), by surrounding at least partially said bone element (3), said ancillary (10) comprising a rod (15) having proximal (19) and distal (17) ends and a longitudinal axis L, the distal end (17) of said rod (15) being provided with a bearing device (21) to bear on the implant (1), said ancillary (10) further comprises a device for tensioning (25) said elongate element (2), **characterized in that** said tensioning device (25) comprises a drum (30) rotatably mounted about an axis T, transverse to the axis L, disposed at the proximal end (19) of the rod (15) and comprising a holding portion (32) adapted to cooperate with the elongate element (2), and **in that** said tensioning device (25) further comprises a device for driving in rotation (34) the drum (30) about the axis T, which makes it possible to tension the elongate element (2) by winding of the elongate element ( 2) around the drum (30), **in that** the driving device (34) comprises a lever (40) pivotally mounted about the axis T, and a clutch device (42) configured so that the pivoting of the lever (40) along the direction of rotation R1 in a first position drives in rotation the drum (30) about the axis T, and **in that** the driving device (34) comprises a clutch-release device (55) configured so that, in the declutched position, the lever (40) being pivoted about the axis T along a direction of rotation R2 opposite to the direction of rotation R1, the drum (30) is freely rotatable about the axis T.

2. The tensioning ancillary (10) according to claim 1, **characterized in that** the driving device (34) comprises a return device (58) configured so that, once the lever (40) is released from the pressure exerted thereon for it to pivot along the direction of rotation R1 in the first position, the lever (40) pivots along a direction of rotation R2, opposite to the direction of rotation R1, in a rest position.

3. The tensioning ancillary (10) according to either one of the claims 1 and 2 , **characterized in that** the driving device (34) comprises a non-return device (70) configured to prevent rotation of the drum (30) along a direction of rotation R2, opposite to the direction of rotation R1, when the lever (40) is in the first position, and optionally in the rest position.

4. The tensioning ancillary (10) according to any one of claims 1 to 3, **characterized in that** the driving device (34) comprises a gripping clamp (80) comprising first (82) and second (84) portions, the first portion (82) being said lever (40) and the second portion (84) being fixed with the rod (15).

5. The tensioning ancillary (10), according to claim 4, **characterized in that** the approximation of the first (82) and second (84) portions of the gripping clamp (80), by pivoting of the first portion (82) forming the lever (40) towards the second portion (84), causes the rotation of the drum (30).

6. The tensioning ancillary (10) according to any one of claims 1 to 5, **characterized in that** the clutch device (42) is a ratchet mechanism (45), particularly comprising a first toothed wheel (46), and optionally a second toothed wheel (48), in connection, rotatably about the axis T, with the drum (30), and a ratchet part (50) in connection with the lever (40).

7. The tensioning ancillary (10) according to claim 6, **characterized in that** the non-return device (70) comprises a ratchet part (71), in particular in connection with the second portion (84) of the clamp (80), configured to cooperate with the first toothed wheel (46), and optionally the second toothed wheel (48).

8. The tensioning ancillary (10) according to any one of claims 1 to 7, **characterized in that** the tensioning device (25) comprises a casing (90) receiving the drum (30), said casing (90) being in fixed connection with the proximal end (19) of the rod (15).

9. The tensioning ancillary (10) according to claim 8, **characterized in that** the drum (30) comprises two lateral ends (31, 33), and **in that** said casing (90) comprises right (91) and left (92) cheeks each having an opening (93) receiving a lateral end (31, 33) of the drum (30).

10. The tensioning ancillary (10) according to claim 9, **characterized in that** the lever (40) comprises a proximal portion (41) and a distal portion (43), said distal portion (43) comprises two lateral projections (44, 49) between which the casing (90) is disposed, and optionally the first (46) and second (48) toothed wheels disposed on either side of the casing (90).

11. The tensioning ancillary (10) according to any one of claims 1 and 6 to 10, **characterized in that** the clutch-release device (55) comprises at least one cam (57, 59), particularly mounted on the casing (90), configured to deactivate the ratchet mechanism (45), particularly to disengage the ratchet part (50) in connection with the lever (40) from the first toothed wheel (46), and optionally from the second wheel toothed (48).

12. The tensioning ancillary (10) according to any one of claims 1 to 11, **characterized in that** the holding portion (32) is a through slot of the drum (30) allowing the passage of at least one free end, preferably both free ends, of the elongate element (2).

13. An assembly **characterized in that** it comprises a tensioning ancillary (10) according to any one of claims 1 to 12, and at least one implant (1, 4), particularly chosen from an implantable longitudinal bar (4) or an implant (1) for holding in tension the elongate element in the wound state on at least one portion of a vertebral body.
